Europäisches Patentamt

European Patent Office

Office européen des brevets

(19)

(11) **EP 0 925 556 B1**

(12) **FASCICULE DE BREVET EUROPEEN**

(45) Date de publication et mention
de la délivrance du brevet:
**05.12.2001 Bulletin 2001/49**

(51) Int Cl.[7]: **G06T 11/00**

(21) Numéro de dépôt: **97938976.4**

(86) Numéro de dépôt international:
**PCT/FR97/01554**

(22) Date de dépôt: **03.09.1997**

(87) Numéro de publication internationale:
**WO 98/10378 (12.03.1998 Gazette 1998/10)**

(54) **PROCEDE DE RECONSTRUCTION D'UNE IMAGE TRIDIMENSIONNELLE D'UN OBJET, EN PARTICULIER UNE IMAGE TRIDIMENSIONNELLE ANGIOGRAPHIQUE**

DREIDIMENSIONALES BILDREKONSTRUKTIONSVERFAHREN, VORZUGSWEISE EINES
DREIDIMENSIONALEN ANGIOGRAPHISCHEN BILDES

METHOD FOR RECONSTRUCTING THE THREE-DIMENSIONAL IMAGE OF AN OBJECT, IN
PARTICULAR A THREE-DIMENSIONAL ANGIOGRAPHIC IMAGE

(84) Etats contractants désignés:
**DE NL**

(30) Priorité: **04.09.1996 FR 9610774**

(43) Date de publication de la demande:
**30.06.1999 Bulletin 1999/26**

(73) Titulaire: **GE MEDICAL SYSTEMS SA**
**78533 Buc Cedex (FR)**

(72) Inventeurs:
• **VAILLANT, Régis**
**F-91140 Villebon sur Yvette (FR)**
• **TROUSSET, Yves**
**F-91120 Palaiseau (FR)**
• **BOUCHERIE, Romain**
**F-92190 Meudon (FR)**
• **ROMEAS, René**
**F-91120 Palaiseau (FR)**

(74) Mandataire: **Goode, Ian Roy et al**
**London Patent Operation**
**General Electric International, Inc.**
**Essex House**
**12-13 Essex Street**
**London WC2R 3AA (GB)**

(56) Documents cités:
• **GARCIA I ET AL: "IMPLEMENTATION AND EXPERIMENTAL EVALUATION OF THE CONSTRAINED ART ALGORITHM ON A MULTICOMPUTER SYSTEM" SIGNAL PROCESSING EUROPEAN JOURNAL DEVOTED TO THE METHODS AND APPLICATIONS OF SIGNAL PROCESSING, vol. 51, no. 1, 1 mai 1996, pages 69-76, XP000625822**
• **HERMAN G T ET AL: "ALGEBRAIC RECONSTRUCTION TECHNIQUES CAN BE MADE COMPUTATIONALLY EFFICIENT" IEEE TRANSACTIONS ON MEDICAL IMAGING, vol. 12, no. 3, 1 septembre 1993, pages 600-609, XP000412338**
• **PAYOT ET AL.: "an adaptative and constrainted model for 3D x-ray vascular reconstruction" PROCEEDINGS OF THE 1995 INTERNATIONAL MEETING ON FULLY THREE-DIMENSIONAL IMAGE RECONSTUCTION IN RADIOLOGY AND NUCLEAR MEDICINE, 4 juillet 1995, FRANCE, pages 19-23, XP000671843**

## Description

L'invention concerne la reconstruction d'une image tridimensionnelle d'un objet à partir d'un ensemble d'images projetées bidimensionnelles dudit objet obtenues pour différentes positions d'un appareil de prises de vues autour de l'objet.

Elle trouve une application particulièrement intéressante dans le domaine médical dans lequel on procède à la reconstruction des structures internes de patient sous examen, en particulier la reconstruction d'images angiographiques c'est-à-dire l'obtention d'images d'arbres vasculaires opacifiés par injection d'un produit de contraste.

L'invention peut trouver néanmoins des applications dans d'autres domaines, notamment dans le contrôle non destructif industriel dans lequel des examens du même type que les examens médicaux sont pratiqués.

Dans le domaine médical, les images projetées bidimensionnelles de l'objet, par exemple la tête d'un patient, sont généralement obtenues par la rotation d'un appareil de prises de vues à rayons X tournant autour de l'objet.

Il existe essentiellement deux types d'algorithmes de reconstruction en imagerie par rayons X.

Un premier type prévoit un calcul de rétroprojection et de filtrage ou encore une reconstruction par transformée de Fourier à plusieurs dimensions.

Un deuxième type, celui qui est concerné par l'invention, concerne les méthodes itératives de reconstruction dites encore algébriques. Le principe d'un tel algorithme algébrique est parfaitement connu de l'homme du métier et a fait déjà l'objet de nombreuses publications. On citera notamment l'article de GORDON, BENDER, et HERMAN intitulé "Algebraic reconstruction technic for three dimensional électron microcopy and X ray photography", Journal THEO. BIOL. 29, pages 471 à 781 (1970), ou bien l'ouvrage de Anil K.JAIN intitulé "Fondamentals of digital image processing" Prentice Hall information and system sciences series, Thomas Kailath Series Edition, ou encore les demandes de brevets français n° 89 03606 ou n° 89 16906.

En bref, après une calibration de l'appareil utilisé pour déterminer notamment les paramètres de la projection dans les plans de projection des images acquises, d'un volume observé décomposé en éléments volumiques élémentaires ou voxels (ces paramètres de calibration formant des matrices de projection), l'algorithme de reconstruction algébrique d'image est utilisé pour reconstruire le volume tridimensionnel à partir de ces images projetées bidimensionnelles. Le principe de base de cet algorithme est d'initialiser les voxels du volume à une valeur initiale prédéterminée, par exemple une valeur nulle, et d'itérer un certain nombre de fois les opérations suivantes : projection des voxels dans le plan de chaque image acquise de façon à obtenir une image virtuelle, détermination de la différence entre le volume projeté (image virtuelle) et l'image acquise correspondante puis rétroprojection de cette différence dans le volume. Après un certain nombre d'itérations, on obtient pour chaque voxel une valeur estimée représentative de la densité de produit de contraste injecté dans les vaisseaux radiographiés ce qui permet de visualiser en trois dimensions la cartographie de ces vaisseaux radiographiés.

Or, un tel algorithme nécessite de déterminer un très grand nombre de fois, typiquement plusieurs millions de fois, la projection d'un voxel en utilisant les différentes matrices de projection.

Si aucune optimisation de l'algorithme n'est effectuée, la détermination de la projection d'un voxel requiert au moins neuf additions, dix multiplications et une division, ce qui est pénalisant pour le temps de calcul du microprocesseur incorporant cet algorithme, et par conséquent pour la durée de reconstruction de l'image.

L'article de Gabor T. Herman intitulé "Algebraic Reconstruction Techniques can be made computationally efficient", IEEE transactions on Medical Imaging 12 1993) September, No. 3, New York US, décrit une mise en oeuvre de techniques de reconstruction d'objet à partir de leurs projections. L'efficacité de cette technique est augmentée en traitant les données d'image dans un ordre prédéterminé, en particulier sur des lignes parallèles à un axe géométrique spécifique de "vue".

Un but de l'invention est d'optimiser le temps de calcul de l'algorithme de reconstruction d'image, et de réduire par conséquent le temps nécessaire pour la reconstruction de l'image tridimensionnelle.

L'invention a ainsi pour but de réduire à une seule addition le nombre d'opérations nécessaires à la détermination de la projection d'un voxel courant connaissant la projection du voxel précédent.

Telle que revendiquée, l'invention propose un procédé de reconstruction d'image tridimensionnelle d'un objet à partir d'un ensemble d'images projetées bidimensionnelles numérisées dudit objet obtenues par différentes positions d'un appareil de prises de vues autour de l'objet, comprenant une calibration de l'appareil dans laquelle on élabore un volume contenant l'objet et décomposé en voxels dont les coordonnées spatiales sont repérées dans un référentiel choisi dit "de calibration", une acquisition dudit ensemble d'images projetées bidimensionnelles numérisées, une application sur chaque image acquise d'un prétraitement, ou rectification, de façon à élaborer une image rectifiée ayant une orientation spatiale prédéterminée et choisie en fonction d'un axe spécifique choisi du référentiel de calibration, par exemple l'axe estimé moyen de rotation de l'appareil autour de l'objet, et une reconstruction de l'image tridimensionnelle comportant l'application d'un algorithme itératif de reconstruction algébrique d'image entre chaque image rectifiée et l'ensemble des voxels en traitant successivement les voxels situés sur des lignes parallèles audit axe spécifique.

On minimise ainsi la durée de reconstruction de l'image tridimensionnelle.

Selon un mode de mise en oeuvre, on acquiert les images projetées bidimensionnelles numérisées à partir de détecteurs placés au niveau de différents plans de projection correspondant aux différentes positions de l'appareil. En pratique ces différentes détections peuvent être obtenues par la rotation d'un seul détecteur autour de l'objet. La calibration de l'appareil fournit notamment des paramètres de calibration (matrices de projection) relatifs à la projection des voxels dans ces plans de projection. Pour la rectification de chaque image bidimensionnelles acquise, on projette l'image bidimensionnelle acquise dans un plan rectifié parallèle audit axe spécifique choisi, on définit dans ce plan rectifié un référentiel rectifié dont l'un des axes est parallèle audit axe spécifique choisi, et on élabore des paramètres de rectification (matrices de rectification) permettant de déterminer les coordonnées de chaque pixel de l'image acquise dans ledit référentiel rectifié associé de façon à élaborer pour l'image acquise ladite image rectifiée numérisée. On modifie alors les paramètres de calibration associés à chaque image acquise compte tenu des paramètres de rectification, par exemple en effectuant un calcul matriciel simple entre les matrices de projection initiales et les matrices de rectification de façon à obtenir des matrices de projection modifiées ou rectifiées. On applique l'algorihme itératif de reconstruction algébrique d'image entre chaque image rectifiée et l'ensemble des voxels compte tenu des paramètres de calibration modifiés en traitement successivement les voxels situés sur des lignes parallèles audit axe spécifique choisi.

En d'autres termes, alors que dans l'art antérieur, on appliquait l'algorithme de reconstruction algébrique d'images directement entre l'ensemble des voxels du volume et l'ensemble des images bidimensionnelles acquises dans les différents plans de projection d'origine, on applique selon l'invention l'algorithme de reconstruction d'images entre des images rectifiées par rapport à ces images acquises et l'ensemble des voxels du volume en traitant successivement les voxels dans un ordre prédéterminé tenant compte du choix de l'axe spécifique du référentiel de calibration.

Plus précisément, on définit à partir du plan de projection d'origine d'une image acquise un plan rectifié parallèle à cet axe spécifique et dans lequel on projette l'image acquise. On repère alors les pixels de l'image rectifiée dans un référentiel de ce plan rectifié dont l'un des axes (l'axe de abscisses ou l'axe des ordonnées) est parallèle audit axe spécifique, la matrice de rectification permettant de déterminer, dans ce référentiel rectifié, les coordonnées de chaque pixel de l'image acquise. La projection des voxels du volume dans le référentiel rectifié de chaque image rectifiée s'effectuera alors à l'aide de la matrice de projection modifiée.

L'invention est remarquable en ce sens que deux des coefficients de cette matrice sont nuls ce qui permet en traitant successivement les voxels dans un ordre prédéterminé de déterminer, connaissant la projection d'un pixel courant, la projection du pixel suivant en n'effectuant qu'une seule addition.

Plus précisément, si z désigne la coordonnée d'un voxel selon l'axe spécifique choisi, par exemple l'axe moyen de rotation de l'appareil, et si x et y désignent les deux autres coordonnées du voxel, on traitera successivement les voxels à x et y constants en faisant varier dans l'ordre la coordonnée z.

D'autres avantages et caractéristiques de l'invention apparaîtront à l'examen de la description détaillée d'un mode de mise en oeuvre nullement limitatif et des dessins annexés sur lesquels :

- la figure 1 illustre schématiquement un jeu d'images projetées bidimensionnelles autour d'un objet,
- la figure 2 illustre plus en détail l'acquisition de l'une de ces images projetées bidimensionnelles et,
- la figure 3 est un organigramme d'un mode de mise en oeuvre du procédé selon l'invention.

Bien que l'invention n'y soit pas limitée on va maintenant décrire l'application du procédé selon l'invention à la reconstruction d'une image angiographique tridimensionnelle d'un patient, en particulier sa tête.

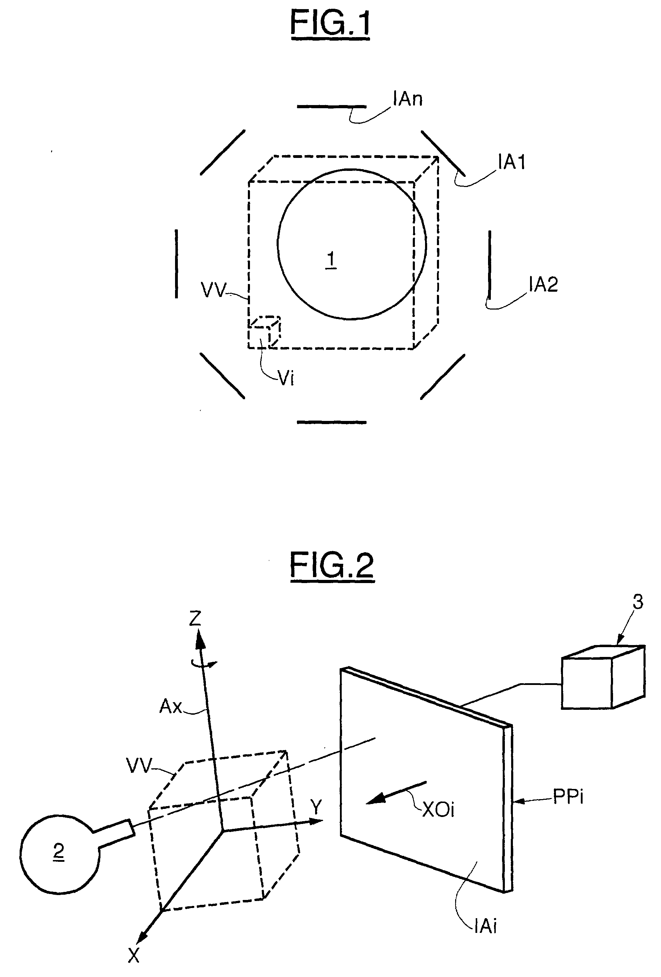

Si l'on se réfère plus particulièrement aux figures 1 et 2, on voit que le système d'imagerie utilisable pour mettre en oeuvre l'invention permet d'obtenir un ensemble d'images acquises bidimensionnelles IA1-IAn obtenues en l'epèce par la rotation autour de la tête 1 d'un patient d'une source 2 à rayons X. En fait, comme il est classique en angiographie, chaque image acquise IAi est une image soustraite qui est par exemple obtenue par une technique classique de soustraction logarithmique de deux radiographies prises sous la même incidence avant et après une injection d'un produit de contraste dans l'arbre vasculaire dont on souhaite reconstruire l'image tridimensionnelle.

Chaque image acquise IAi est obtenue à partir d'un détecteur de rayonnement bidimensionnel, par exemple du type amplificateur de luminance utilisé en radiologie, disposé en vis-à-vis du tube à rayons X dans un plan dit plan de projection PPi. Les différents plans de projection sont obtenus par les différentes positions angulaires du détecteur en rotation autour de la tête du patient. La normale XOi au plan de projection PPi définit l'axe optique de l'image acquise IAi. Le détecteur est relié à des moyens de traitement 3 comportant notamment des moyens d'échantillonnage reliés à un microprocesseur incorporant de façon logicielle dans sa mémoire de programme associé l'algorithme de reconstruction algébrique d'images utilisé dans l'invention et d'une façon générale tous les moyens fonctionnels permettant la mise en oeuvre du procédé selon l'invention.

Dans le cas d'un système d'imagerie à rayons X composé d'une source de rayons X et d'un détecteur bidimensionnel, l'opération géométrique intervenant dans la production de l'image acquise est une projection conique d'un objet analysé, déployé dans un espace à trois dimensions, sur un espace à deux dimensions qui est celui du plan de projection correspondant au plan de détection. Les paramètres géométriques décrivant complètement les diverses projections coniques doivent être connus. Or, il est souvent impossible et trop imprécis, d'accéder à ces paramètres de façon directe, c'est-à-dire, par exemple, en mesurant directement sur le système d'acquisition la distance entre la source de rayons X et le détecteur.

On appelle calibration d'un système d'imagerie, l'opération qui aboutit à la connaissance indirecte précise des paramètres géométriques qui interviennent dans la production d'une image. Le principe, classique et connu, est basé sur l'utilisation d'un fantôme géométrique connu dans l'espace tridimensionnel, et dont on acquiert la projection bidimensionnelle. Plus précisément, la calibration comporte les étapes suivantes :

- on dispose d'un objet connu, le fantôme de calibration, présentant un certain nombre de points caractéristiques dont la position dans l'espace est connue par des coordonnées mesurées par rapport à un repère propre à cet objet;
- on acquiert l'image de ce fantôme dans les conditions géométriques d'un point de vue (ou incidence) que l'on veut calibrer;
- on reconnaît les projections des points caractéristiques dans l'image. Pour cela on associe chaque point caractéristique de l'objet à sa trace dans l'image acquise projetée;
- on inverse, au sens mathématique, le système d'équation décrivant la projection;
- et on obtient finalement l'ensemble des paramètres de la projection pour le point de vue donné.

Une forme de fantôme de calibration géométrique souvent utilisée est celle d'un cube, aux huit coins duquel sont disposées des billes métalliques opaques aux rayons X. La calibration étant une opération connue de l'homme de métier, elle ne sera pas décrite plus en détails, d'autant que plusieurs publications ont déjà décrit le principe d'une calibration géométrique manuelle. On peut notamment citer les articles suivant :

(1) D.L. Parker, J.Wu, D.L Pope, R. Van Bree, G.R. Caputp and H.W. Marshall, "Three-dimensional reconstruction and flow measurements of coronary arteries using multi view digital angiography", in New Developmnts in Quantitative Coronary Arterography, J.C. Reiber and P.W. Serruys Eds., pp. 225-247, Kluwer Academic Publishers, 1988;
(2) D.J. Hawks, A.C.F. Colchester and C.R. Mol; "The accurate 3-D reconstruction of the geometric configuration of the vascular trees from X-ray recordings," in Physics and Engineering of Medical Imaging, R. Guzzardi Ed., Nijhoff, 1987.

(3) M. Garreau, J-L Coatrieux, R. Collorec and C. Chardenon, "A Knowledge-based approach for 3-d reconstruction and labeling of vascular networks from biplane angiographic projections", IEEE Medical Imaging, vol. 10, n°2, pp. 122-131, June 1991.

Il est également possible d'utiliser un procédé connu de calibration géométrique automatique d'un système d'imagerie par rayons X, tel que celui décrit dans la demande de brevet français n° 93 00804. En bref, on utilise pour une telle calibration automatique un fantôme dans lequel les billes sont distribuées, de proche en proche, en une succession telle que des altitudes de billes, mesurées le long de l'axe de rotation du système d'imagerie, et surtout un axe du fantôme, soient monotones croissantes (ou décroissantes) avec un numéro d'ordre des billes dans la succession.

La calibration du système d'imagerie permet notamment de déterminer l'axe moyen estimé Ax de rotation de l'appareil de prises de vues autour de l'objet ainsi que la position de la source 2 et les caractéristiques géométriques des axes optiques des différentes images acquises. La calibration permet également de définir un volume virtuel VV (intersection des différents cônes de projection) entourant l'objet 1 et décomposé en éléments volumiques élémentaires Vi ou "voxels". Ce volume VV, et donc chaque voxel Vi, est spatialement repéré dans un référentiel, dénommé ci-après référentiel de calibration, dont l'un des axes, en l'espèce l'axe Z, est confondu avec l'axe de rotation estimé Ax. Il convient de noter ici que les plans de projection PPi dans lesquels se projettent les images acquises IAi ne sont généralement pas parallèles à l'axe Z.

La calibration permet également de définir pour chaque image acquise IAi une matrice de projection Pi permettant de déterminer, pour chaque voxel Vi les coordonnées de sa projection (pixel) dans l'image acquise IAi correspondante.

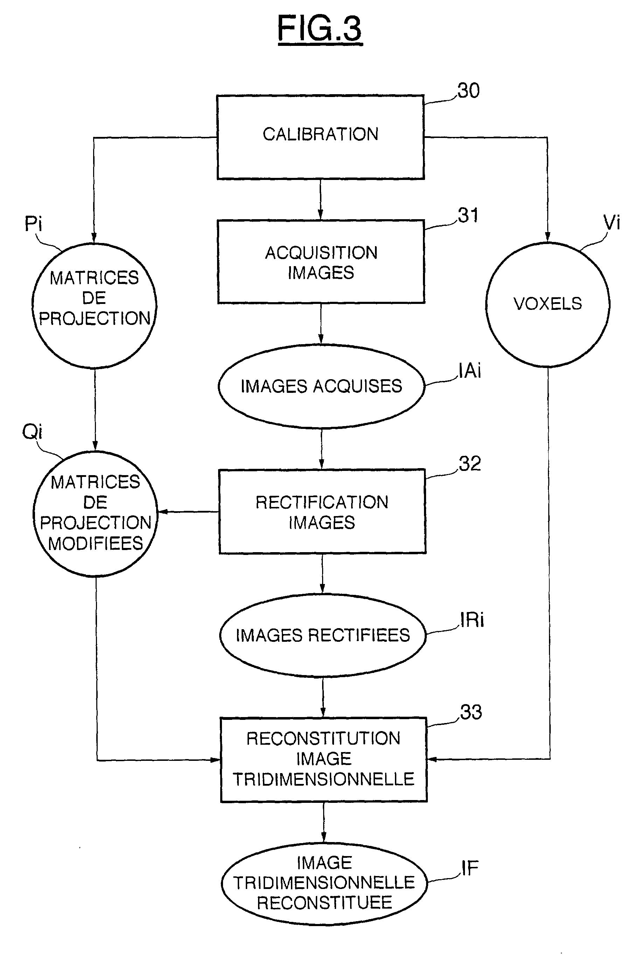

On va maintenant décrire en se référant plus particulièrement à la figure 3 un mode de mise en oeuvre du procédé selon l'invention. Alors que selon l'art antérieur, l'algorithme de reconstruction algébrique d'image était appliqué directement sur les images acquises IAi (obtenues après calibration 30 et acquisition 31) un élément essentiel de l'invention consiste ici à effectuer sur les images acquises un prétraitement ou rectification 32 de façon à obtenir des images rectifiées IRi sur lesquelles on effectuera la reconstruction de l'image tridimensionnelle.

Plus précisément, pour obtenir l'image rectifiée IRi à partir de l'image acquise IAi correspondante, on projette l'axe optique XOi du plan de projection PPi de l'image acquise IAi dans un plan orthogonal à l'axe Z puis on définit le plan de l'image rectifiée comme étant le plan orthogonal à la projection de cet axe optique XOi. Le plan rectifié dans lequel va se trouver l'image rectifiée est donc parallèle à l'axe Z. On définit ensuite dans ce plan rectifié un référentiel rectifié dont l'un des axes, par exemple l'axe des abscisses, est parallèle à l'axe Z.

On définit alors une matrice de rectification permettant de déterminer les coordonnées de chaque pixel de l'image acquise IAi dans ce référentiel rectifié. Les nouvelles coordonnées de ces pixels définissent ainsi une image rectifiée numérisée IRi. Les calculs élémentaires de transformation géométriques des projections permettant de passer d'une image acquise à une image rectifiée et d'élaborer la matrice de rectification correspondante, sont des calculs classiques pour l'homme du métier et n'ont pas été décrits plus en détail ci-avant à des fins de simplification. L'ensemble de ces opérations est effectué de façon logicielle dans le microprocesseur des moyens de traitement.

A partir de cette matrice de rectification, on élabore une matrice de projection modifiée Qi obtenue simplement par une multiplication à gauche de la matrice de projection Pi (obtenue après calibration) par la matrice de rectification.

La matrice Qi de projection modifiée permet donc de déterminer les coordonnées de la projection de chaque voxel Vi dans le référentiel rectifié de l'image rectifié et de déterminer en conséquence si ce voxel se projette effectivement ou non dans cette image rectifiée.

Cette matrice de projection modifiée Qi est de la forme :

$$\begin{pmatrix} I_{11} & I_{12} & I_{13} & I_{14} \\ I_{21} & I_{22} & 0 & I_{24} \\ I_{31} & I_{32} & 0 & I_{34} \end{pmatrix}$$

On remarque donc que deux des coefficients de cette matrice Qi, à savoir les coefficients $I_{23}$ et $I_{33}$, sont nuls.

L'étape suivante du procédé consiste à opérer la reconstruction 33 de l'image tridimentionnelle IF en appliquant l'algorithme de reconstruction algébrique d'image entre l'ensemble des images rectifiées IRi et l'ensemble des voxels Vi du volume VV.

A cet égard, on affecte à chaque voxel Vi un coefficient $\mu$ que l'on initialise à une valeur initiale, en général zéro. A l'issue de l'étape de reconstruction, c'est-à-dire après un nombre prédéterminé d'itérations de l'algorithme de reconstruction d'image, on obtiendra un ensemble de valeurs pour tous les coefficients $\mu$ associés à tous les voxels du volume. Chaque coefficient $\mu$ est représentatif de la position du voxel correspondant par rapport à un vaisseau sanguin. En effet, si la valeur $\mu$ affectée à un voxel est nulle, alors ce voxel ne se situe pas dans un vaisseau sanguin tandis qu'une valeur non nulle de ce coefficient signifie que le voxel correspondant se situe dans un vaisseau sanguin. Il sera donc possible, à l'aide d'un dispositif de visualisation classique, de reconstituer visuellement l'image tridimensionnelle de l'arbre vasculaire situé dans la tête du patient à partir de l'ensemble des valeurs des coefficients $\mu$ obtenues.

Dans chaque itération de l'algorithme de reconstruction d'image, on considère successivement chaque image rectifiée IRi.

Pour chaque image IRi, on détermine tout d'abord pour l'ensemble des voxels du volume, les coordonnées de la projection de chaque voxel Vi dans le référentiel rectifié de l'image IRi à l'aide de la matrice de projection modifiée Qi. Si le voxel Vi se projette effectivement dans l'image IRi, c'est-à-dire si les coordonnées du pixel projeté correspondant se situent à l'intérieur des limites de l'image rectifiée IRi, on affecte alors à ce pixel projeté un niveau de gris égal au niveau de gris de ce pixel obtenu lors de l'itération précédente, augmenté de la valeur courante du coefficient $\mu$ associé au voxel Vi. On définit ainsi une image virtuelle IVi à laquelle on soustrait l'image réelle rectifiée IRi. On obtient alors une image de résidu dont chacun des pixels est affecté d'un niveau de gris de résidu. On effectue alors une rétroprojection ou projection inverse, de cette image de résidu dans le volume VV. En pratique pour cette rétroprojection, on effectue sur chaque voxel Vi du volume VV les mêmes calculs de projection que ceux effectués dans l'étape de projection précédente mais, cette fois-ci, on modifie la valeur du coefficient $\mu$ associé à un voxel Vi qui se projette effectivement dans l'image de résidu en ajoutant à la valeur $\mu$ actuelle du voxel, la valeur de résidu du pixel correspondant, à un coefficient de normalisation près.

Une fois que ces opérations de projection-rétroprojection ont été effectuées pour l'ensemble des voxels pour une image rectifiée IRi, on effectue les mêmes opérations pour l'image rectifiée suivante et on réitère l'ensemble de ces opérations un certain nombre de fois, en pratique trois.

Un autre élément essentiel de l'invention, en combinaison avec le prétraitement des images acquises ayant permis d'obtenir les images rectifiées, consiste à traiter successivement les voxels Vi dans un ordre prédéterminé.

Plus précisément, si x, y et z désignent les coordonnées d'un voxel Vi, et si u et v désignent les coordonnées de la projection de ce voxel Vi dans le référentiel rectifié de l'image rectifée correspondante, les coordonnées u et v sont obtenues par les formules suivantes :

$$u = \frac{\lambda_1}{\lambda_3} \qquad v = \frac{\lambda_2}{\lambda_3}$$

où $\lambda_1$, $\lambda_2$ et $\lambda_3$ sont définis par les formules suivantes :

$$\lambda_1 = I_{11}\, x + I_{12}\, y + I_{13}\, z + I_{14}$$

$$\lambda_2 = I_{22}\, x + I_{23}\, y + \qquad I_{24}$$

$$\lambda_2 = I_{32}\, x + I_{33}\, y + \qquad I_{34}$$

compte tenu du fait que deux des cofficients de la matrice de projection modifiée Qi sont nuls.

Aussi, si l'on cherche à déterminer les coordonnées de la projection du voxel suivant, de coordonnées (x, y, z+1), les coefficients $\lambda_1^n$, $\lambda_2^n$ et $\lambda_3^n$ affectés à ce voxel suivant sont définis par les formules :

$$\lambda_1^{\,n} = \lambda_1 + I_{13}$$

$$\lambda_2^{\,n} = \lambda_2$$

$$\lambda_3^{\,n} = \lambda_3$$

Il en résulte donc que les coordonnées $u^n$ et $v^n$ de la projection de ce voxel suivant sont définies par les formules :

$$u^n = u + \frac{I_{13}}{\lambda_3}$$

$$v^n = v$$

L'homme du métier remarque donc que puisque $I_{13}$ et $\lambda_3$ sont constants, la détermination de la coordonnée $u^n$ ne nécessite d'effectuer qu'une seule addition d'une constante à la coordonnée u connue et préalablement stockée.

En d'autres termes, on traitera les voxels groupe par groupe, tous les voxels d'un même groupe ayant leur couple de coordonnées (x, y) constant, et à l'intérieur d'un groupe, on fera varier successivement la troisième coordonnée du voxel. En d'autres termes, ceci revient à traiter successivement les voxels situés sur les lignes parallèles à l'axe z. Sur une même ligne de traitement le calcul de la projection du voxel courant connaissant la projection du voxel précédent ne nécessitera qu'une seule addition. Bien entendu, des opérations supplémentaires sont toutefois nécessaires à chaque changement de ligne de traitement, c'est-à-dire lorsqu'on modifie l'une ou l'autre des deux autres coordonnées x et y d'un voxel.

## Revendications

1. Procédé de reconstruction d'une image tridimensionnelle d'un objet à partir d'un ensemble d'images projetées bidimensionnelles numérisées dudit objet obtenues par différentes positions d'un appareil de prises de vues autour de l'objet, comprenant une calibration (30) de l'appareil dans laquelle on élabore un volume (VV) contenant l'objet (1) et décomposé en voxels (Vi) dont les coordonnées spatiales sont repérées dans un référentiel choisi dit de calibration, une acquisition (31) dudit ensemble d'images projetées bidimensionnelles numérisées (IAi), une application sur chaque image acquise (IAi) d'un prétraitement (32) de façon à élaborer une image rectifiée (IRi) ayant une orientation spatiale prédéterminée et choisie en fonction d'un axe spécifique choisi (Ax) du référentiel de calibration et une reconstruction (33) de l'image tridimensionnelle (IF) comportant l'application d'un algorithme itératif de reconstruction algébrique d'image entre chaque image rectifiée (IRi) et l'ensemble des voxels (Vi) en traitant successivement les voxels situés sur des lignes parallèles audit axe spécifique, ce qui permet de minimiser la durée de reconstruction de l'image tridimensionnelle.

2. Procédé selon la revendication 1, **caractérisé par le fait qu'**on acquiert les images projetées bidimensionnelles numérisées (IAi) à partir de détecteurs placés au niveau de différents plans de projection (PPi) correspondant aux différentes positions de l'appareil, la calibration de l'appareil fournissant des paramètres de calibration (Pi) relatifs à la projection des voxels dans ces plans de projection , **par le fait que** dans le prétraitement (32) de chaque image bidimensionnelle acquise, on projette l'image bidimensionnelle acquise dans un plan rectifié parallèle audit axe spécifique choisi, on définit dans ce plan rectifié un référentiel rectifié dont l'un des axes est parallèle audit axe spécifique choisi (Ax), et on élabore des paramètres de rectification permettant de déterminer les coordonnées de chaque pixel de l'image acquise dans ledit référentiel rectifié associé de façon à élaborer pour l'image acquise ladite image rectifiée numérisée, **par le fait qu'**on modifie les paramètres de calibration (Pi) associés à chaque image acquise compte tenu des paramètres de rectification, et on applique (33) l'algorithme itératif de reconstruction algébrique d'image entre chaque image rectifiée (IRi) et l'ensemble des voxels (Vi) compte tenu des paramètres de calibration modifiés (Qi) en traitant successivement les voxels situés sur des lignes parallèles audit axe spécifique choisi (Ax).

3. Procédé selon la revendication 2, **caractérisé par le fait que** l'axe optique (XOi) de chaque image acquise (IAi) étant déterminé lors de la calibration de l'appareil, on détermine le plan rectifié d'une image acquise comme étant le plan orthogonal à la projection de l'axe optique de cette image acquise dans le plan orthogonal audit axe spécifique choisi (Ax).

**4.** Procédé selon l'une des revendications précédentes, **caractérisé par le fait que**, l'appareil effectuant une rotation autour de l'objet, la calibration de l'appareil détermine l'axe estimé moyen (Ax) de rotation de l'appareil, et **par le fait qu'**on choisit cet axe moyen de rotation (Ax) comme axe spécifique (Z) du référentiel de calibration.

**5.** Procédé selon l'une des revendications précédentes, pour la reconstruction d'une image angiographique tridimensionnelle, **caractérisé par le fait que** l'acquisition de chaque image projetée bidimensionnelle comporte la différence de deux images de l'objet obtenues, avant et après injection d'un produit de contraste dans l'objet, pour une même position de l'appareil.

**Patentansprüche**

**1.** Verfahren zur Rekonstruktion eines drei-dimensionalen Bildes eines Objektes ausgehend von einer Gesamtheit von zweidimensionalen, nummerierten Projektionsbildern des Objekts, die von verschiedenen um das Objekt herumliegenden Positionen einer Bildaufnahmevorrichtung erhalten wurden, und das die folgenden Schritte umfasst: eine Kalibrierung (30) der Vorrichtung, bei der ein Volumen (VV) erstellt wird, welches das Objekt (1) beinhaltet und unterteilt ist in Voxels (Vi), deren räumliche Koordinaten in einem ausgewählten sogenannten Kalibrierungsbezugssystem ausgedrückt sind; eine Aufnahme (31) der Gesamtheit der zweidimensionalen nummerierten Projektionsbilder (IAi); eine Anwendung einer Vorverarbearbeitung (32) auf jedes der aufgenommenen Projektionsbilder (IAi), um ein korrigiertes Bild (IRi) zu erzeugen, das eine vorbestimmte räumliche Orientierung hat, die abhängig von einer ausgewählten spezifischen Achse (Ax) des Kalibrierungsbezugssystems gewählt wurde; und eine Rekonstruktion (33) des drei-dimensionalen Bildes (IF), die die Anwendung eines iterativen Algorithmus zur algebraischen Bildrekonstruktion zwischen jedem korrigierten Bild (IRi) und der Gesamtheit der Voxels (Vi) umfaßt, wobei sukzessive die Voxels behandelt werden, die auf zu der spezifischen Achse parallelen Linien liegen, wodurch es möglich wird, die Dauer der Rekonstruktion des drei-dimensionalen Bildes zu minimieren.

**2.** Verfahren nach dem Anspruch 1, **dadurch gekennzeichnet dass** die zwei-dimensionalen Projektionsbilder (IAi) von Detektoren aufgenommen werden, die auf dem Niveau verschiedener, mit den verschiedenen Positionen der Vorrichtung korrespondierenden Projektionsebenen (PPi) angeordnet sind; dass die Kalibrierung der Vorrichtung Kalibrationsparameter (Pi) bezüglich der Projektion der Voxels in diesen Projektionsebenen liefert; dadurch dass bei der Vorverarbeitung (32) jedes aufgenommenen zweidimensionalen Bildes das aufgenommene zweidimensionale Bild in eine zu der ausgewählten spezifischen Achse parallele, korrigierte Ebene projeziert wird; dass in dieser korrigierten Ebene ein korrigiertes Bezugssystem definiert wird, dessen eine Achse parallel zu der ausgewählten, spezifischen Achse (Ax) liegt, und dass Korrekturparameter ermittelt werden, die es ermöglichen, die Koordinaten jedes Bildpunktes des aufgenommenen Bildes in dem zugeordneten korrigierten Bezugssystem zu bestimmen, wobei für das aufgenommene Bild das nummerierte, korrigierte Bild ermittelt wird; dadurch dass die jedem aufgenommenen Bild zugeordneten Kalibrationsparameter (Pi) unter Berücksichtigung der Korrekturparameter geändert werden, und dass der iterative Algorithmus zur algebraischen Bildrekonstruktion zwischen jedem korrigierten Bild (IRi) und der Gesamtheit der Voxel (Vi) unter Berücksichtiging der gänderten Kalibrationsparameter (Qi) angewendet wird, wobei sukzessive die Voxel behandelt werden, die auf zu der spezifischen Achse parallelen Linien liegen.

**3.** Verfahren nach dem Anspruch 2, **dadurch gekennzeichnet dass**, weil die optische Achse (XOi) jedes aufgenommenen Bildes (IAi) während der Kalibration der Vorrichtung bestimmt wird, die korrigierte Ebene des aufgenommenen Bildes so bestimmt wird als ob es die Ebene wäre, die orthogonal zur Projektion der optischen Achse dieses orthogonal zu besagter ausgewählter spezifischer Achse (Ax) aufgenommen Bildes ist.

**4.** Verfahren nach einem der vorgehenden Ansprüche, **dadurch gekennzeichnet dass**, weil die Vorrichtung eine Rotation um das Objekt ausführt, die Kalibrierung der Vorrichtung die geschätzte mittlere Rotationsachse (Ax) der Vorrichtung bestimmt, und dadurch dass diese mittlere Rotationsachse (Ax) als spezifische Bezugsachse (Z) des Kalibrierungsbezugssystems gewählt wird.

**5.** Verfahren nach einem der vorhergehenden Ansprüche für die Rekonstruktion eines angiographischen dreidimensionalen Bildes, **dadurch gekennzeichnet dass** die Aufnahme jedes zwei-dimensionalen Projektionsbildes die Differenz von zwei Bildern des Objektes umfaßt, die vor und nach Injektion eines Kontrastmittels in das Objekt bei gleicher Position der Vorrichtung aufgenommen wurden.

## Claims

1. Process for reconstructing a three-dimensional image of an object from a set of digitized two-dimensional projected images of the said object obtained for various positions of a picture-taking apparatus around the object, comprising a calibration (30) of the apparatus, a capture (31) of the said set of digitized two-dimensional projected images (IAi), and a reconstruction (33) of the three-dimensional image (IF) from the captured projected two-dimensional images and from an iterative algebraic image reconstruction algorithm, **characterized in that** in the calibration step a volume (W) is computed containing the object (1) and decomposed into voxels (Vi) whose spatial coordinates are labelled in a chosen so-called calibration reference frame, a preprocessing (32) is applied to each captured image (IAi) so as to compute a corrected image (IRi) having a predetermined and chosen spatial orientation dependent on a chosen specific axis (Ax) of the calibration reference frame, and the iterative algebraic image reconstruction algorithm is applied between each corrected image (IRi) and the set of voxels (Vi), processing the voxels in succession in a predetermined order linked with the choice of the said specific axis, this making it possible to minimize the duration of reconstruction of the three-dimensional image.

2. Process according to Claim 1, **characterized in that** the digitized two-dimensional projected images (IAi) are captured using detectors placed level with various projection planes (PPi) corresponding to the various positions of the apparatus, calibration of the apparatus providing calibration parameters (Pi) relating to the projection of the voxels in these projection planes, **in that** in the preprocessing (32) of each captured two-dimensional image, the captured two-dimensional image is projected into a corrected plane parallel to the said chosen specific axis, a corrected reference frame, one of whose axes is parallel to the said chosen specific axis (Ax), is defined in this corrected plane, and correction parameters are computed which make it possible to determine the coordinates of each pixel of the captured image in the said associated corrected reference frame so as to compute the said digitized corrected image for the captured image, **in that** the calibration parameters (Pi) associated with each captured image are modified having regard to the correction parameters, and the iterative algebraic image reconstruction algorithm is applied (33) between each corrected image (IRi) and the set of voxels (Vi) having regard to the modified calibration parameters (Qi), processing in succession the voxels located on lines parallel to the said chosen specific axis (Ax).

3. Process according to Claim 2, **characterized in that** the optical axis (XOi) of each captured image (IAi) being determined during the calibration of the apparatus, the corrected plane of a captured image is determined as being the plane orthogonal to the projection of the optical axis of this captured image in the plane orthogonal to the said chosen specific axis (Ax).

4. Process according to one of the preceding claims, **characterized in that**, the apparatus performing a rotation about the object, the calibration of the apparatus determines the mean estimated axis (Ax) of rotation of the apparatus, and **in that** this mean axis of rotation (Ax) is chosen as specific axis (Z) of the calibration reference frame.

5. Process according to one of the preceding claims, for the reconstruction of a three-dimensional angiographic image, **characterized in that** the capturing of each two-dimensional projected image includes the differencing of two obtained images of the object, before and after injecting a contrast medium into the object, for one and the same position of the apparatus.

# FIG.1

# FIG.2

# FIG.3